# EUROPEAN PATENT SPECIFICATION

(11) **EP 3 795 162 B1**
(45) Date of publication and mention of the grant of the patent: **23.08.2023**
(21) Application number: 20196411.1
(22) Date of filing: 16.09.2020
(51) Int. Cl.: A61K 36/04, A61K 36/48, A61P 1/16

(54) **HERBAL PREPARATION FOR THE TREATMENT OF FATTY LIVER DISEASE**
PFLANZLICHE ZUSAMMENSETZUNG ZUR BEHANDLUNG VON FETTLEBERERKRANKUNGEN
COMPOSITION À BASE D'HERBES POUR LE TRAITEMENT DE LA STÉATOSE HÉPATIQUE

(30) Priority: 17.09.2019 IT 201900016520
(43) Date of publication of application: 24.03.2021
(73) Proprietor: Phytoitalia SRL, 20124 Milano (IT)
(72) Inventor: ABBIATI, Ezio, 20011 Corbetta (MI) (IT)
(74) Representative: Rastelli, Franco

(56) References cited:
- WO-A1-2013/166563
- IT-A1- UB20 155 978
- IT-A1- UB20 155 979
- IT-A1- UB20 155 985
- LUCA IMPERATORI ET AL: "Feasibility single-arm study of a medical device containing Desmodium adscendens and Lithothamnium calcareum combined with chemotherapy in head and neck cancer patients", CANCER MANAGEMENT AND RESEARCH, vol. Volume 10, 1 January 2018 (2018-01-01), pages 5433-5438, XP055694247, GB ISSN: 1179-1322, DOI: 10.2147/CMAR.S165746
- Anonymous: "Autoriz. a condurre lo Studio di fase II "DESMOVIT" presso la UOC Oncologia PO "Santa Croce" Fano -CE14127-PARERE UNICO", , 23 October 2014 (2014-10-23), XP055272195, Retrieved from the Internet: URL:http://www.albopretorionline.it/marche nord/download.aspx?ida=308522&pubb=1&n=1 [retrieved on 2016-05-12]
- MAGIELSE JOANNA ET AL: "Antihepatotoxic activity of a quantified Desmodium adscendens decoction and d-pinitol against chemically-induced liver damage in rats", JOURNAL OF ETHNOPHARMACOLOGY, ELSEVIER IRELAND LTD, IE, vol. 146, no. 1, 3 January 2013 (2013-01-03), pages 250-256, XP028981012, ISSN: 0378-8741, DOI: 10.1016/J.JEP.2012.12.039
- FRANÇOIS CÉLINE ET AL: "Safety of Desmodium adscendens extract on hepatocytes and renal cells. Protective effect against oxidative stress", JOURNAL OF INTERCULTURAL ETHNOPHARMACOLOGYUNITED STATES,, vol. 4, no. 1, 31 December 2014 (2014-12-31), pages 1-5, XP009511961, ISSN: 2146-8397, DOI: 10.5455/JICE.20141013041312

## Description

### BACKGROUND OF THE INVENTION

The present invention relates to a herbal preparation, particularly effective for the treatment of fatty liver disease or hepatic steatosis.

Hepatic steatosis, also known by the acronym NAFLD (Non-Alcoholic Fatty Liver Disease) refers to a spectrum of histological liver disorders, characterized by a hyperaccumulation of intrahepatocytic fat (steatosis, consisting of a cellular accumulation of triglycerides of more than 5%), with no significant alcohol consumption or secondary causes of liver disease.

Traditionally, simple steatosis has a negligible risk of progression to cirrhosis. However, a significant percentage of individuals with NAFLD (10-15%) have histological necroinflammation aspects and balloon-like degeneration characterizing the most severe form of liver disease, i.e. non-alcoholic steatohepatitis (NASH), which may progress to fibrosis, cirrhosis and related complications, including hepatocellular carcinoma (HCC).

From the aetiopathogenetic point of view, NAFLD is a complex and multi-factorial disease, the onset and severity of which depend upon a combination of genetic and environmental factors. Even though, on the one hand, it is considered the hepatic expression of the metabolic syndrome (visceral obesity, glucidic intolerance and type 2 diabetes mellitus, dyslipidemia and hypertension), on the other, the primary, parallel and independent role of NAFLD in the onset of the syndrome and in the development of its complications has been demonstrated.

The progressive and ubiquitous change in lifestyle and eating habits over recent decades has significantly influenced the epidemiological aspects of this disease. NASH has become a significant clinical problem, not only from the gas-troenterological point of view, as it is destined to become the primary cause of cirrhosis and HCC, but also in the endocrinology and internal medicine fields as an independent risk factor for cardiovascular disease and dysmetabolic complications.

Considering the high prevalence of NAFLD in the general population and its potential clinical implications, early and reliable identification of high-risk individuals appears to be of primary importance, in order to monitor and prospectively predict the onset of complications that affect their hepatic and extra-hepatic prognosis.

For this reason, the method currently considered the most reliable to ascertain the state of hepatic steatosis is Fibroscan, which is essentially an ultrasound system that sends elastic waves to the liver, through a probe placed on the chest wall in the intercostal spaces. The speed of propagation of these waves through the liver tissue is processed by a computer, which provides a real-time quantitative estimate of the degree of elasticity and stiffness of the liver.

The first line of therapy, recommended in all patients with NAFLD and also aimed at limiting the cardiovascular risk, is therefore non-pharmacological. Weight loss, which can be pursued through changes in lifestyle (increased physical activity and healthier eating habits) favors an improvement in liver histology when it is greater than 7-10% of the initial weight. In a study conducted on 261 patients with NAFLD, treated through diet and physical activity for 12 months and subjected to biopsy at the beginning and the end of treatment, the weight loss was proportional to the histological regression of NASH (25% of total cases) without worsening of fibrosis. Individually adapted pragmatic approaches that combine dietary restriction with a progressive increase in aerobic and endurance exercises are preferable.

It can therefore be concluded that the current therapy for hepatic steatosis is limited to the control of the patient's lifestyle, including daily physical activity.

There is currently no scientific evidence to guide the treating professional towards the use of Desmodium in the treatment of hepatic steatosis, which, as explained above, is currently limited to controlling the patient's lifestyle.

The publication "Feasibility single-arm study of a medical device containing Desmodium adscendens and Lithothamnium calcareum combined with chemotherapy in head and neck cancer patients", Vol. 10, 1 January 2018 (2018-01-01), pages 5433-5438, XP055694247, GB ISSN: 1179-1322, DOI: 10.2147/CMAR.S165746, concludes that chemotherapy, combined with Desmodium and Lithothamnium, improved pain and fatigue in head and neck cancer patients.

### SUMMARY OF THE INVENTION

The main aim of the present invention is to provide an aid in the treatment of hepatic steatosis which, when combined with changes in the patient's lifestyle, significantly improves the latter's state of health.

This and other aims are achieved through the herbal preparation of claim 1. Preferred embodiments of the invention will be apparent from the remaining claims.

In relation to the known methods for treating hepatic steatosis, the invention offers the advantage of restoring the physiological function of the fatty liver through the consumption of a herbal preparation containing Desmodium adscendens (common name Desmodium).

### BRIEF DESCRIPTION OF THE DRAWINGS

Figure 1 is a Fibroscan graphical representation of the data presented in Table 1.
Figure 2 is a Fibroscan graphical representation of the data presented in Table 2.

### DESCRIPTION OF THE PREFERRED EMBODIMENTS

The inventor has now surprisingly discovered that, contrary to the current trend of treating fatty liver exclusively by making changes to the patient's lifestyle, oral treatment with Desmodium reduces hepatic steatosis by up to 30% more than such lifestyle changes alone.

Desmodium is a plant of African origin, known for its anti-inflammatory action on the bronchi and its effectiveness in the treatment of allergic asthma.

In particular, the present invention consists of a herbal preparation that is based on pulverized Desmodium and can be administered orally. For its administration to humans, the herbal preparation of the invention is preferably administered in the form of a food supplement, together with commonly used carriers and excipients.

For oral compositions, solid forms, such as capsules, tablets, granules and powders, or liquid forms, such as suspensions or solutions, can be prepared. The compositions of the invention preferably take a solid form, advantageously in the form of edible gelatin capsules. Other types of galenic solutions for administering the herbal preparation, such as syrups, tablets or sachets for the powder preparation, are also clearly possible.

The herbal preparation of the invention preferably takes the form of capsules each containing 100 to 300 mg of Desmodium adscendens, preferably between 200 and 300 mg of Desmodium adscendens.

The quantity of desmodium that has proved to be most effective in treating hepatic steatosis according to the invention ranges from 800 mg to 1.2 g per day, preferably 1.2 g per day. According to the invention, use is made of the leaves, that is, the aerial parts of Desmodium, dried and cryoground (ground in a cryogenic mill).

Use is preferably made of Lithothamnium calcareum as an excipient, which, due to its magnesium content, exhibits a desired anti-caking action, thus avoiding the use of other potentially hepatotoxic substances.

Other excipients suitable for the invention include anti-caking agents based on silicon, lactose, magnesium stearate and the like.

The components Desmodium and Lithothamnium calcareum are advantageously present in the herbal preparation of the invention in an amount of 70% to 90% by weight of Desmodium and 30% to 10% by weight of Lithothamnium calcareum.

In order to investigate the therapeutic potential of the herbal preparation of the invention for the treatment of hepatic steatosis, the following study was conducted.

### Experimental part

### Example 1

Coated capsules are prepared, each one containing 350 mg of the herbal preparation containing:
300 mg of Desmodium
50 mg of Mg stearate

### Example 2

Coated capsules are prepared, each one containing 350 mg of herbal preparation with:
300 mg of Desmodium and 50 mg of Lithothamnium calcareum
310 mg of Desmodium and 40 mg of Lithothamnium calcareum
290 mg of Desmodium and 60 mg of Lithothamnium calcareum

### Study inclusion criteria

- Fibroscan at baseline T0 (first visit);
- absence of previously diagnosed diseases, such as: diabetes, autoimmune diseases, specific endocrine disorders and rheumatic diseases;
- clinical biochemistry tests at times T0 and T1 (first and second visits) on the following parameters: GOT, GPT, γGT, ferritin, complete blood count with formula, triglycerides, Tot / HDL / LDL cholesterol, cholinesterase, albumin, direct, indirect and total bilirubin.

### Patients

Twenty-one men and women patients aged between 29 and 73 years were treated. All patients were kept on a diet that included 1.2 g / day of the Desmodium-based herbal preparation of the invention for a period of four months (120 days).

### Results

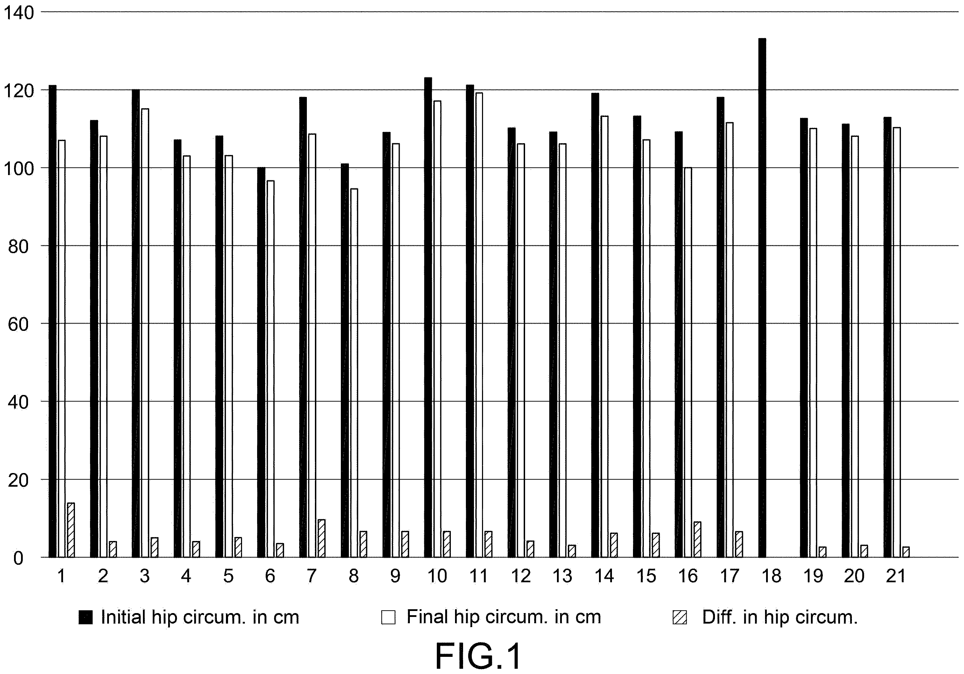

The results of the treatment are shown in Table 1 below and in Figure 1 (only the initial data of patient 18 are included as he dropped out of the treatment, for this reason Table 2 does not show the results for this patient), wherein it is shown how the diet influenced the weight insignificantly as an average weight loss of less than 8% of the weight at the beginning of the therapy was obtained with a decrease in BMI (Body Mass Index) following a similar trend but, despite the limited weight losses, an excellent reduction in hepatic steatosis was recorded (Fibroscan data) with an average reduction of 18% and this, on the basis of the studies mentioned above, confirms that even with a weight reduction of less than 10%, an improvement of steatosis was obtained and that this was not given by the diet (weight loss less than 10%) but by the use of the herbal preparation based on Desmodium and Lithothamnium calcareum:

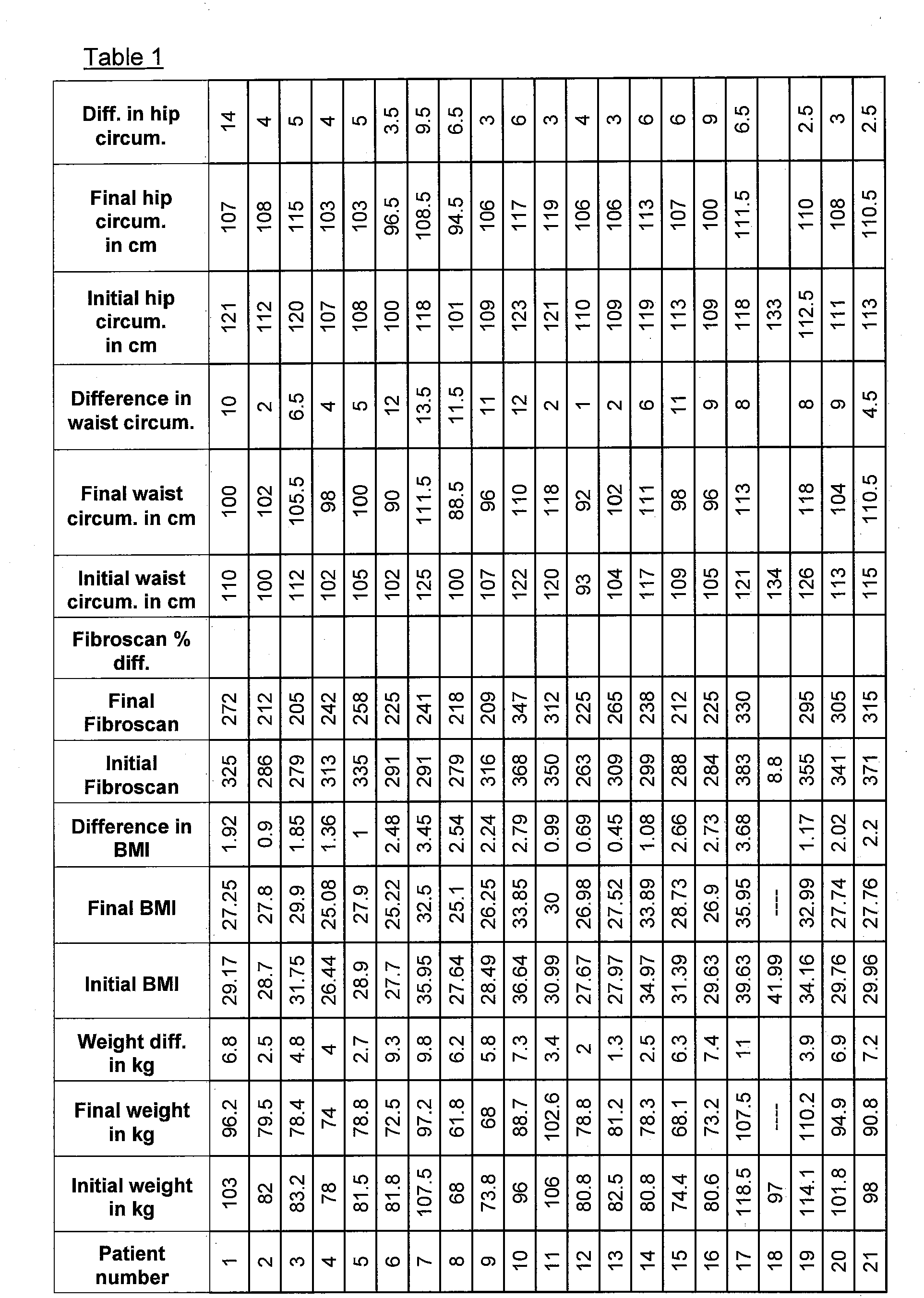

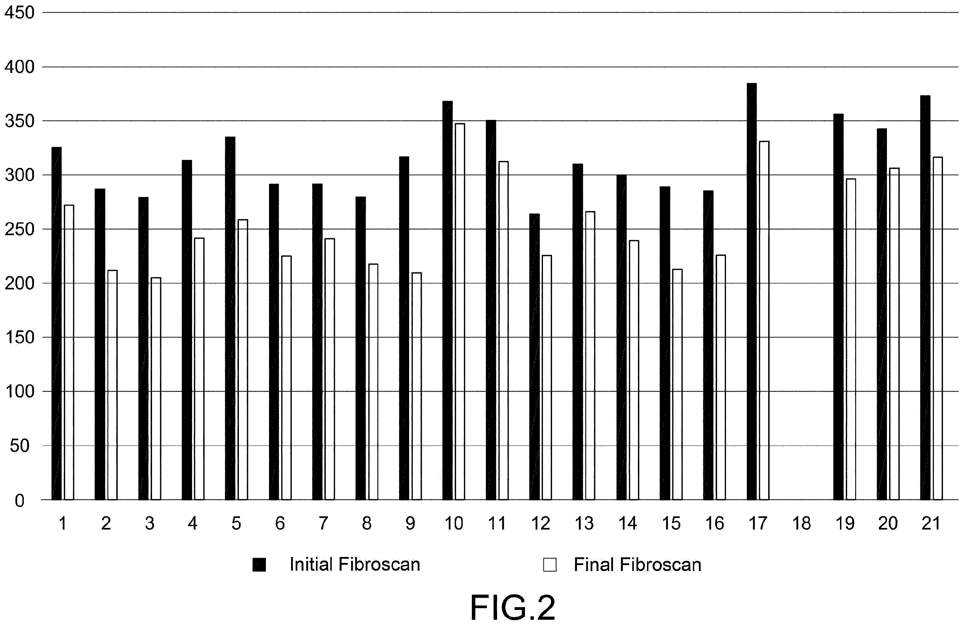

The Fibroscan values are also shown in Table 2 below and in Figure 2, from which it can be seen that, despite the diet having little influence on weight reduction and the BMI, Desmodium and Lithothamnium calcareum significantly reduced the hepatic steatosis, which, in some cases, disappeared altogether (values below 253 db/m):

**Table 2**

| **Initial Fibroscan** | **Final Fibroscan** | **Difference in Fibroscan** |
|---|---|---|
| 325 | 272 | 16.30769 |
| 286 | 212 | 25.87413 |
| 279 | 205 | 26.5233 |
| 313 | 242 | 22.68371 |
| 335 | 258 | 22.98507 |
| 291 | 225 | 22.68041 |
| 291 | 241 | 17.18213 |
| 279 | 218 | 21.8638 |
| 316 | 209 | 33.86076 |
| 368 | 347 | 5.706522 |
| 350 | 312 | 10.85714 |
| 263 | 225 | 14.44867 |
| 309 | 265 | 14.23948 |
| 299 | 238 | 20.40134 |
| 288 | 212 | 26.38889 |
| 284 | 225 | 20.77465 |
| 383 | 330 | 13.83812 |
| 355 | 295 | 16.90141 |
| 341 | 305 | 10.55718 |
| 371 | 315 | 15.09434 |

which shows
S1 > 253db/m (steatosis > 10%) 92%
S2 > 285db/m (steatosis > 33%) 92%
S3 > 310db/m (steatosis > 67%) 88%

In other words, the results of the Fibroscan analyses (see also graph in Figure 2) show that, compared to the decrease in weight (maximum 10% and often even less), an improvement ranging from 15 to 30% is obtained, therefore higher than reported in the studies already published on the subject (for example, *"*Non-alcoholic fatty liver disease (NAFLD)" by Bugianesi and Marietti - II Pensiero Scientifico Editore, 07 Jun 2019, published in https://www.ncbi.nlm.nih.gov/pubmed/27571466

### Conclusions

On the basis of the results obtained from 4 observations at 1 month, 2 months, 3 months and 4 months, it was seen that the improvement of hepatic steatosis, which, with diet and lifestyle changes alone leads to an improvement in liver elasticity measured on the Fibroscan only if a weight loss of more than 10% is obtained, reached a value of up to 40% higher on the 21 patients also receiving the herbal preparation. In addition to this, there was also a slight improvement in other indices, such as the BMI and triglyceride levels.

## Claims

1. A herbal preparation for use in the treatment of hepatic steatosis, **characterized in that** it consists of an oral formulation containing Desmodium adscendens as an extract of leaves, stems or roots.

2. A preparation for use according to claim 1, **characterized in that** it consists of an oral formulation containing 800 mg to 1.2 g of Desmodium adscendens for daily administration.

3. A preparation for use according to claim 1, **characterized in that** it is in the form of capsules, each one containing 100 to 300 mg of Desmodium adscendens.

4. A preparation for use according to claim 1, **characterized in that** it is in the form of capsules, each one containing 200 to 300 mg of Desmodium adscendens.

5. A preparation for use according to claim 1, **characterized in that** it also comprises Lithothamnium calcareum as an extract of leaves, stems or roots.

6. A preparation for use according to claim 5, **characterized in that** it is in the form of tablets containing 70% to 90% by weight of Desmodium adscendens and 30% to 10% by weight of Lithothamnium calcareum.

7. A food supplement comprising a preparation according to claims 1 to 6 for use according to one or more of claims 1 to 6, in a solid form for oral administration.

## Patentansprüche

1. Kräuterpräparat zur Verwendung bei der Behandlung von hepatischer Steatose, **dadurch gekennzeichnet, dass** es aus einer oralen Formulierung besteht, die Desmodium adscendens als Extrakt von Blättern, Stielen oder Wurzeln enthält.

2. Präparat zur Verwendung nach Anspruch 1, **dadurch gekennzeichnet, dass** es aus einer oralen Formulierung besteht, die 800 mg bis 1,2 g Desmodium adscendens zur täglichen Verabreichung enthält.

3. Präparat zur Verwendung nach Anspruch 1, **dadurch gekennzeichnet, dass** es in Form von Kapseln vorliegt, wobei jede 100 bis 300 mg Desmodium adscendens enthält.

4. Präparat zur Verwendung nach Anspruch 1, **dadurch gekennzeichnet, dass** es in Form von Kapseln vorliegt, wobei jede 200 bis 300 mg Desmodium adscendens enthält.

5. Präparat zur Verwendung nach Anspruch 1, **dadurch gekennzeichnet, dass** es auch Lithothamnium calcareum als Extrakt von Blättern, Stielen oder Wurzeln umfasst.

6. Präparat zur Verwendung nach Anspruch 5, **dadurch gekennzeichnet, dass** es in Form von Tabletten vorliegt, die 70 bis 90 Gew. -% Desmodium adscendens und 30 bis 10 Gew. -% Lithothamnium calcareum enthalten.

7. Nahrungsergänzungsmittel umfassend ein Präparat nach den Ansprüchen 1 bis 6 zur Verwendung nach einem oder mehreren der Ansprüche 1 bis 6, in einer festen Form, für die orale Verabreichung.

## Revendications

1. Préparation à base d'herbes pour son utilisation dans le traitement de la stéatose hépatique, **caractérisée en ce qu'**elle est constituée d'une formulation orale contenant Desmodium adscendens sous la forme d'un extrait de feuilles, de tiges, ou de racines.

2. Préparation pour son utilisation selon la revendication 1, **caractérisée en ce qu'**elle est constituée d'une formulation orale contenant de 800 mg à 1,2 g de Desmodium adscendens pour une administration quotidienne.

3. Préparation pour son utilisation selon la revendication 1, **caractérisée en ce qu'**elle est sous la forme de capsules, chacune contenant de 100 à 300 mg de Desmodium adscendens.

4. Préparation pour son utilisation selon la revendication 1, **caractérisée en ce qu'**elle est sous la forme de capsules, chacune contenant de 200 à 300 mg de Desmodium adscendens.

5. Préparation pour son utilisation selon la revendication 1, **caractérisée en ce qu'**elle comprend également Lithothamnium calcareum sous la forme d'un extrait de feuilles, de tiges ou de racines.

6. Préparation pour son utilisation selon la revendication 5, **caractérisée en ce qu'**elle est sous la forme de comprimés contenant de 70% à 90% en poids de Desmodium adscendens et de 30% à 10% en poids de Lithothamnium calcareum.

7. Supplément alimentaire comprenant une préparation selon les revendications 1 à 6 pour son utilisation selon une ou plusieurs des revendications 1 à 6, sous une forme solide pour une administration orale.
